# EUROPEAN PATENT APPLICATION

(11) **EP 1 770 139 A1**
(43) Date of publication of application: **04.04.2007**
(21) Application number: 05256043.0
(22) Date of filing: 28.09.2005
(51) Int. Cl.: C09K 3/00

(54) **Ultraviolet light absorber and its use**

(71) Applicant: KABUSHIKI KAISHA KAISUI KAGAKU KENKYUJO, Kitakyushu-shi, Fukuoka-ken (JP)
(72) Inventor: Miyata, Shigeo, Kabushiki Kaisha Kaisui Kagaku, Kitakyushu-shi Fukuoka-ken (JP)
(74) Representative: Srinivasan, Ravi Chandran

(57) **Abstract**

An ultraviolet absorber containing, as active ingredient, a zinc oxide solid solution of the formula (1),

(Zn)₁₋ₓM³⁺_{x-δ}O·nSiO₂ (1)

wherein M³⁺ is at least one trivalent metal selected from Al³⁺, Fe³⁺ and Ce³⁺, 0 < x < 0.2, 0.001 < n < 0.2 and δ is a cationic lattice defect,
in which solid solution the surface of a crystal is chemically bonded to a silicic acid,
which solid solution has an average secondary particle diameter of 1.5 µm or less and a BET specific surface area of at least 40 m²/g.

## Description

### Technical Field of the Invention

The present invention relates to an ultraviolet absorber containing as an active ingredient a zinc oxide solid solution which is improved in water resistance, ultraviolet absorbability and heat resistance (decreased in photocatalytic activity), a process for the production thereof, a sun block cosmetic material containing the above ultraviolet absorber and an ultraviolet absorbable resin composition containing the above ultraviolet absorber.

### Prior Arts of the Invention

As anultraviolet absorber, there are organic compound ultraviolet absorbers such as benzotriazole type and benzophenone type ultraviolet absorbers and inorganic compound ultraviolet absorbers such as titanium oxide and zinc oxide. In recent years, a demand for safety (nontoxic to low toxic) is increasing for ultraviolet absorbers. Therefore, an ultraviolet absorber which is safe and excellent in ultraviolet absorbability and also has high transparency or high whiteness is desired. For this reason, organic ultraviolet absorbers which are apt to be colored in yellow, that has a fear of toxicity, are not suitable for fields of cosmetics or food-packaging materials (mainly, plastics), where high safety is required, in particular food-packaging materials which have a possibility of entering a mouth.

In this respect, ultrafine-particle titanium oxide and zinc oxide, which come on the market recently, have high safety and are improved in transparency so that they receive attention. However, the ultrafine-particle titanium oxide has a high refractive index. For this reason, although it is formed of ultrafine particles, it is still poor in transparency and is also poor in ultraviolet absorbability in UVA region (320 - 400 nm) . On the other hand, the ultrafine-particle zinc oxide has a lower refractive index than titanium oxide so that it has high transparency. However, the transparency is still insufficient. In addition, the ultrafine-particle zinc oxide has strong hygroscopicity and there is a problem (photocatalytic action) that the ultrafine-particle zinc oxide deteriorates a resin with heat or light.

Hence, the present inventor has found that a zinc oxide solid solution which has an average secondary particle diameter of from 0.1 to 1.5 µm and a BET specific surface area of at least 20 m²/g and is represented by the formula (2),

(Zn)₁₋ₓM³⁺_{x-δ}O (2)

wherein M³⁺ is a trivalent metal selected from Al, Fe and Ce, x is in the range of 0 < x < 0.2 and δ is a cationic lattice defect,
is well-balanced and excellent in all of safety, ultraviolet absorbability and transparency (JP-A-2000-144095).

It is found that the zinc oxide solid solution represented by the formula (2) has the following defect. When the zinc oxide solid solution represented by the formula (2) is exposed to air, it comes to easily absorb moisture in the air as the BET specific surface area increases, in other words as transparency increases. Accordingly, the zinc oxide solid solution represented by the formula (2) gradually converts into a hydroxide from the surface thereof. In this case, the ultraviolet absorbability and transparency gradually decrease. Further it is also found that there is another defect that, since the above zinc oxide solid solution shows photocatalytic action, it decreases the mechanical strength of a resin with heat or light.

### Summary of the Invention

It is an object of the present invention to provide a safe ultraviolet absorber containing as a main component a zinc oxide solid solution which is excellent in transparency, ultraviolet absorbability and water resistance and is decreased in photocatalytic activity, a sun block cosmetic material containing the above ultraviolet absorber and a resin composition containing the above ultraviolet absorber such as a food-packaging material.

In particular, it is another object of the present invention to provide a resin type packaging material for food and electric materials which packaging material is capable of inhibiting or preventing deterioration of food or electric materials because of ultraviolet rays.

It is further another object of the present invention to improve decrease the photocatalytic action of zinc oxide and improve the heat resistance and light resistance of a resin, etc.

According to the present invention, there is provided an ultraviolet absorber containing, as an active ingredient, a zinc oxide solid solution of which the surface of a crystal is chemically bonded to a silicic acid (SiO₂) and which is represented by the formula (1),

(Zn)₁₋ₓM³⁺_{x-δ}O·nSiO₂ (1)

wherein M³⁺ is at least one trivalent metal selected from A1, Fe and Ce, x is in the range of 0 < x < 0.2, preferably 0.05 < x ≤ 0.12, n is in the range of 0.001 < n < 0.2, preferably 0.005 ≤ x ≤ 0.1, and δ is a cationic lattice defect, and has an average secondary particle diameter of 1.5 µm or less and a BET specific surface area of at least 40 m²/g, preferably an average secondary particle diameter of 1.0 µm or less and a BET specific surface area of at least 50 m²/g, particularly preferably an average secondary particle diameter of 0.5 µm or less and a BET specific surface area of at least 60 m²/g.

According to the present invention, there is further provided an ultraviolet absorber according to the above, wherein M³⁺ is Al or a combination of Al and Fe or Al and Ce.

According to the present invention, there is still further provided a process for the production of the above ultraviolet absorber, which process comprises carrying out coprecipitation of a mixed aqueous solution of a water-soluble salt of Zn and a water-soluble salt of at least one trivalent metal selected from Al, Fe and Ce with maintaining a pH value of at least 6 with an alkali aqueous solution to obtain a coprecipitate, mixing the coprecipitate with a silicic acid or a silicic acid precursor before or after washing the coprecipitate with water or an alkali metal carbonate aqueous solution, drying the mixture, and firing the dried mixture at 300 °C or higher.

According to the present invention, there is furthermore provided a sun block cosmetic material containing as an active ingredient the above zinc oxide solid solution, of which the surface of a crystal is chemically bonded to a silicic acid. The zinc oxide solid solution is preferably surface-treatment with perfluoroalkyl phosphate ester and/or a silicone oil.

According to the present invention, there is further provided an ultraviolet absorbable resin composition containing 100 parts by weight of a resin and 0.01 to 10 parts by weight of a zinc oxide solid solution of which the surface of a crystal is chemically bonded to a silicic acid and which is represented by the formula (1),

(Zn)₁₋ₓM³⁺_{x-δ}O·nSiO₂ (1)

wherein M³⁺ is at least one trivalent metal selected from Al, Fe and Ce, x is in the range of 0 < x < 0.2, preferably 0.05<x≤0.12, n is in the range of 0.001 < n < 0.2, preferably 0.005 ≤ x ≤ 0.1, and δ is a cationic lattice defect, and has an average secondary particle diameter of 1.5 µm or less and a BET specific surface area of at least 40 m²/g, preferably an average secondary particle diameter of 1.0 µm or less and a BET specific surface area of at least 50 m²/g, particularly preferably an average secondary particle diameter of 0.5 µm or less and a BET specific surface area of at least 60 m²/g.

According to the present invention, it is possible to improve defects of a zinc oxide solid solution having fine particles and having a high specific surface area, which defects are water resistance and thermal stability due to photocatalytic action, and also improve transparency and ultraviolet absorbability.

### Detailed Description of the Invention

Thepresent inventor has found that chemicallybonding the surface of a zinc oxide solid solution represented by the formula (2) to a silicic acid (oxide of Si) improves water resistance, inhibits growth of primary particles at firing and improves ultraviolet absorbability and transparency. Further, the present inventor has found that chemical bonding of an electron-donative zinc oxide solid solution (basic) to an electron-accepting silicic acid (acid) can reduce the photocatalytic action of the zinc oxide solid solution and, for example, can inhibit deterioration of a resin due to heat or light.

It is preferred for obtaining excellent transparency, whiteness and ultraviolet absorbability that the zinc oxide solid solution of which the surface of a crystal is chemically bonded to a silicic acid used in the present invention has as high a BET specific surface area as possible and as small a secondary particle diameter, obtained by a particle size distribution measurement, as possible. Specifically, the BET specific surface area is 40 m²/g or more, preferably 50 m²/g or more, particularly preferably 60 m²/g or more. The average secondary particle diameter is 1.5 µm or less, preferably 1.0 µm or less, particularly preferably 0.5 µm or less.

The chemical bonding of the crystal surface to the silicic acid in the present invention is achieved by carrying out coprecipitation of a mixed aqueous solution of water-soluble salts of Zn and M³⁺ with maintaining a pH value of 6 or higher with an aqueous solution of alkali such as sodium hydroxide to obtain a coprecipitate, then, after or before washing the coprecipitate with water or a water-soluble carbonic acid alkali such as sodium carbonate, adding and mixing a silicic acid or a silicic acidprecursor to/with the coprecipitate in an aqueous medium with stirring to allow the silicic acid or the silicic acid precursor to adsorb to or react with the coprecipitate, carrying out treatment (s) properly selected from washing with water, filtration, drying and pulverization, and then firing the resultant material at 300 °C or higher, preferably from 300 to 600 °C. Examples of the silicic acid or silicic acid precursor include sodium silicate (water glass No. 3, water glass No.1, sodium orthosilicate), colloidal silica and organic silicon compounds which convert into a silica (SiO₂) component after hydrolysis or after firing at about 300 °C or higher, such as ethyl silicate, methyl silicate and a silane coupling agent. Owing to the firing at 300 °C or higher, metal (Zn or Al) of the surface of the solid solution reacts with the silicic acid, whereby the surface of the solid solution converts into a metal silicate and thus water resistance appears. At the same time, the silicic acid inhibits growth of primary particles. The range of the amount n of the silicic acid component used is 0.001 < n < 0.2, preferably 0.005 ≤ n ≤ 0.1, particularly preferably 0.01 ≤ n ≤ 0.06. When the amount of the SiO₂ component is smaller than the lower limit of the above range, the effect of improving water resistance and the inhibition of growth of primary particles are insufficient. When it is larger than the upper limit of the above range, the metal silicate undergoes crystallization and ultraviolet absorbability decreases.

Examples of the water-soluble salts of Zn and M³⁺ used in the production of the ultraviolet absorber of the present invention include water-soluble zinc salts such as zinc chloride, zinc nitrate and zinc sulfate and water-soluble salts of M³⁺ such as aluminum chloride, aluminum nitrate, aluminum sulfate, ferric chloride and cerium chloride.

For obtaining fine secondary particles and increasing transparency, it is preferred to dry-mill the above coprecipitate with a pulverizing mill such as a jet mill, to wet-mill the coprecipitate in an aqueous medium (ball mill pulverization) or to wet-mill the coprecipitate in an organic medium after the firing. The diameter of balls used is preferably, for example, from about 1 mm to 0.2 mm.

In the zinc oxide solid solution represented by the formula (1) used in the present invention, at least one metal selected from Al, Fe and Ce can be used as M³⁺. It is preferred to use Al alone, a combination of Al and Fe or a combination of Al and Ce in view of whiteness, price, etc. The range of x, which represents the proportion of M³⁺, is 0 < x < 0.2, preferably 0.03 ≤ x ≤ 0.15, particularly preferably 0.05 ≤ x ≤ 0.12. When x is too large, ultraviolet absorbability decreases. When x is too small, transparency tends to decrease.

The ultraviolet absorber of the present invention may be surface-treated with a surface-treating agent. The surface-treated ultraviolet absorber is suited for use in a sun block cosmetic material, a packaging material made of a resin, and the like. Examples of the surface-treating agent include silicones such as methyl hydrodiene polysiloxane and methyl polysiloxane; silane coupling agents such as n-hexyltrimethoxysilane; fluorine-containing phosphate esters such as perfluoroalkyl phosphate ester; phosphate esters such as stearyl acid phosphate; higher fatty acids such as stearic acid; metal salts of fatty acids such as sodium stearate and zinc stearate; amino acids such as N-acylglutamic acid; an ester of a copolymer of 2-ethylhexyl acrylate, methacrylic acid, methyl methacrylate and butyl methacrylate, with a silicone obtained by substituting part of methyl group of methyl polysiloxane with hydroxyl group; and nonionic surfactants such as sorbitanmonooleate. The amount of the surface-treating agent used is 0.1 to 10 % based on the weight of the ultraviolet absorber of the present invention. The surface-treatment can be carried out by a common wet or dry process.

The sun block cosmetic material containing the ultraviolet absorber of the present invention as an active ingredient may further contains an additive in addition to the zinc oxide solid solution of the present invention. Examples of the additive include antioxidants such as vitamin C, vitamin E and catechin, a melamine-generation inhibitor such as Flavosterone, aloe extract and burnet extract, moisturizing agents such as hyaluronic acid, an amino acid derivative (extract extracted from sugar beet), sorbitol, glycerin, propylene glycol, chondroitin sulfate and dl pyrrolidone carboxylic acid, whitening agents such as tea extract, scutellaria root extract and glycyrrhiza extract, antibacterial agents such as burnet extract and eucalyptus extract, antiinflammatory agents such as horse chestnut, antiallergic agents such as glycyrrhizin, emollient agents such as squalane oil, olive oil, lanolin and lecithin, ultraviolet scattering agents such as talc, mica and scattering agents obtained by carrying titanium oxide or zinc oxide on the surface of talc or mica, emulsifying agents such as polyacrylic acid amide, and dispersing agents such as a silicone oil and C₆ to C₁₂ alkyl trimethicone.

Examples of the resin used in the present invention include thermoplastic resins such as polyethylene, polypropylene, polystyrene, ABS, polyacrylate, polycarbonate, polyethylene terephthalate, polymethacrylate, polyamide, polyester, an ethylene vinyl acetate copolymer, polymethylpentene, polybutene, polyvinyl chloride and polyvinyl acetate; thermosetting resins such as a phenol resin, a melamine resin, an epoxy resin, an unsaturated polyester resin and an alkyd resin; rubbers such as EPDM, SBR, NBR, butyl rubber, isoprene rubber and chlorosulfonated polyethylene; and synthetic fibers such as an acrylic fiber, an acetate fiber, nylon, a polyester fiber, a polypropylene fiber, a polyethylene fiber and a polyurethane fiber. However, the resin is not limited to these examples.

The method of mixing and kneading the resin and the ultraviolet absorber of the present invention is not specially limited. Any mixing means may be adopted, so long as the means can uniformly mix both the components. For example, there may be used an extruder, a roll, a Banbury mixer or a homogenizer. No special limitation is also imposed upon the molding method. For example, it includes an injection molding, an extrusion molding, a blow molding, a press molding, a rotation molding, a calender molding, a sheet forming molding, a vacuum molding and spinning.

The resin composition of the present invention which is transparent and is excellent in ultraviolet absorbability and a packaging material for food, etc., comprising the above resin composition may further contain a variety of additives as required. Examples of the additives include an anti-oxidant, a lubricant, an antistatic agent, a pigment, a foaming agent, a plasticizer, a filler, a reinforcing agent, a crosslinker, an antibacterial agent, a mildewproofing agent and an anti-adhesion agent.

### Examples

The present invention will be explained more in detail with reference to Examples, hereinafter.

### Example 1

A mixed solution of aluminum chloride and zinc chloride (Zn = 1.5 mol/l, Al=0.1mol/l) at a flow rate of 100 ml/minute and a sodium hydroxide aqueous solution having a concentration of 4 mol/l at a flow rate of about 80 ml/minute were added with ameteringpump into a 5-liter-volume reaction vessel containing about 1 liter of water with stirring while the flow rate of the sodium hydroxide aqueous solution was adjusted so as to maintain a pH value at 9 to 9.4 (temperature: about 35 - 37 °C), thereby obtaining a coprecipitate. The coprecipitate was washed with a sodium carbonate aqueous solution having a concentration of 0.2 mol/l until an AgNO₃ aqueous solution did not become cloudy precipitation of AgCl in a filtrate. Then, it was washed with water. The washed coprecipitate was dispersed in water, to prepare 10 liters of a slurry having a solid (coprecipitate) concentration of about 150 g/l. Water glass No. 3 (sodium silicate) having an Na₂O concentration of 0. 1 mol/l was added in an amount of 30 g calculated as SiO₂ with stirring. The resultant mixture was uniformly blended and then dried with a spray dryer. The thus-obtained dried material was pulverized with a counter jetmill (supplied by HOSOKAWA Micron Corporation) and then fired with an electric furnace at 380 °C for 1 hour. The fired material showed an X-ray diffraction pattern of zinc oxide alone, while it was slightly shifted to a high angle side. Therefore it was found that the fired material was a solid solution of Al in ZnO, and it was also found that the silicic acid component was chemically bonded to Zn and/or Al and was in amorphous form. The fired material had a BET specific surface area of 54 m²/g, measured by a liquid nitrogen adsorption method. The firedmaterial was measured for a particle size distribution with a laser diffraction method particle size distribution measuring device after a pre-treatment. The above pre-treatment was carried out by applying ultrasonic waves to disperse the fired material in isopropyl alcohol for 5 minutes. According to the particle size distribution measurement, the average secondary particle diameter at a cumulation of 50 % was 0.52 µm and the average secondary particle diameter at a cumulation of 90 % was 1. 20 µm. The amount of SiO₂ was measured by a method in which the fired material was dissolved under heat in a mixed solution of hydrochloric acid and perchloric acid , filtration was carried out, Zn and Al in a filtrate were chelated, insoluble contents were washed, then water-soluble contents were placed in a platinum crucible and fired at 800 °C for 1 hour, and then the weight thereof was measured. As a result thereof, the chemical constitution was as follows. Zn_{0.94}Al_{0.06}O·0.03SiO₂

### Example 2

Example 1 was repeated except that the amount of the water glass No.3, calculated as SiO₂, in Example 1 was changed to 50 g. The X-ray diffraction of firedmaterial was a diffraction pattern which corresponded to zinc oxide, while it was slightly shifted to a high angle side. Further, aweakdiffractionpattern which was supposed to be silicate was present at spacing of about 2 Å. The BET specific surface area was 59 m²/g. The average secondary particle diameter at a cumulation of 50 % was 0.54 µm and the average secondary particle diameter at a cumulation of 90 % was 1.27 µm. The chemical constitution was as follows. Zn_{0.94}Al_{0.06}0-0.05SiO₂

### Comparative Example 1

Example 1 was repeated except that the water glass No.3 treatment carried out in Example 1 was not carried out. The X-ray diffraction pattern of fired material was similar to that in Example 1. The BET specific surface area was 37 m²/g. The average secondary particle diameter at a cumulation of 50 % was 0.63 µm and the average secondary particle diameter at a cumulation of 90 % was 1.30 µm. The chemical constitution was as follows. Zn_{0.94}Al_{0.06}O

### Example 3

A slurry of a coprecipitate was prepared in the same manner as in Example 1 except that the mixed aqueous solution used in Example 1 was replaced with a mixed solution of zinc chloride and aluminum nitrate (Zn²⁺ = 1.0 mol/l, Al = 0.1 mol/l). Ethyl silicate (having a concentration of 28 % as SiO₂) in an amount of 6 g, calculated as SiO₂, was added to 4 liters of the above slurry (150 g/l calculated as a solid) with stirring. The mixture was stirred for about 20 minutes, hydrolysis was carried out and surface treatment was carried out. The resultant material was spray-dried and then pulverized with an atomizer and the pulverized material was fired in an electric furnace at 460 °C for 1 hour. 200 g of the fired material was dispersed in 1,000 ml of isopropyl alcohol. The resultant dispersion together with 1 liter of glass beads having a diameter of 0.5 mm was placed in a 3-liter-volume ball mill and wet-milled for 15 hours. Then, the wet-milled material was dried at about 100 °C for 10 hours and then pulverized with an atomizer. The X-ray diffraction pattern of the pulverized material was similar to that of ZnO, while it was slightly shifted to a high angle side. The BET specific surface area was 64 m²/g. The average secondary particle diameter at a cumulation of 50 % was 0.20 µm and the average secondary particle diameter at a cumulation of 90 % was 0.38 µm. The chemical constitution was as follows. Zn_{0.91}Al_{0.09}O·0.15SiO₂

### [Water (moisture absorption) resistance Test]

Each of powders of the silicic acid-treated zinc oxide solid solutions of the present invention obtained in Examples 1, 2 and 3 and the zinc oxide solid solution, which was not treated with silicic acid, obtained in Comparative Example 1 was independently weighed in an amount of 5 g. These powders were respectively placed in Schale having a diameter of 5 cm and they were exposed and stored at room temperature for 7 days. These powders were each weighed after 7 days, to measure moisture-absorption amounts respectively. Table 1 shows the results thereof.

**Table 1**

| Sample | Moisture-absorption amount (%) |
|---|---|
| Solid solution prepared by the method of Example 1 | 1.1 |
| Solid solution prepared by the method of Example 2 | 1.2 |
| Solid solution prepared by the method of Example 3 | 0.9 |
| Solid solution prepared by the method of Comparative Example 1 | 10.2 |

### [Measurement results of visible light and ultraviolet light transmittances]

One of the zinc oxide solid solutions of the present invention obtained in Examples 1, 2 and 3, the ZnO type solid solution obtained in Comparative Example 1, zinc oxide No.1 having a BET specific surface area of 9m²/g (Comparative Example 2) and ultrafine-particle titanium oxide having a BET specific surface area of 60 m²/g (Comparative Example 3) was mixed in an amount of 1 % with a linear low density polyethylene (LLDPE) . The mixture was kneaded with an open roll at 170 °C for 10 minutes and then molded with a press molding device to obtain a sheet having a thickness of 0.3 mm. The sheet was measured for visible light and ultraviolet light transmittances in wavelength of 200 to 500 nm with a spectrophotometer. The transmittance of visible light was evaluated at 500 nm and the transmittance of ultraviolet light was evaluated at 360 nm and 280 nm. Table 2 shows the results.

**Table 2**

| Light transmittance (%) of LLDPE (thickness 0.3 mm) containing 1 % of ultraviolet absorber | | | | | | |
|---|---|---|---|---|---|---|
| Ultraviolet absorber | Ex.1 | Ex.2 | Ex.3 | CEx.1 | CEx.2 | CEx.3 |
| 500 nm | 92.9 | 94.2 | 95.7 | 90.7 | 75.8 | 82.4 |
| 360 nm | 1.2 | 1.4 | 0.7 | 4.5 | 4.9 | 13.4 |
| 280 nm | 1.0 | 1.1 | 0.2 | 4.2 | 9.4 | 3.2 |

| | | | | | | |
|---|---|---|---|---|---|---|
| Ex. = Example, CEx. = Comparative Example | | | | | | |

From the above results, it is found that the zinc oxide solid solutions of which the surface of a crystal was chemically bonded to a silicic acid, provided by the present invention, were improved in both transparency and ultraviolet absorbability as compared with the zinc oxide solid solution and zinc oxide which were not treated with silicic acid.

### [Thermal stability test]

One of the zinc oxide solid solution (Example 1), which was treated with silicic acid, of the present invention and the zinc oxide solid solution (Comparative Example 1), which was not treated with silicic acid, was mixed in an amount of 2 % by weight with polypropylene, and the mixture was molten and kneaded with a twin-screw extruder at about 220 to 230 °C, to obtain a pellet. The pellet was molded with an injection molding machine at about 230 °C to prepare a board having a thickness of 2 mm, a length of 150 m and a width of 80 mm. The board was placed in an oven at 150 °C and subjected to a 20-days thermal stability test. Then, the board was checked for a weight residual rate and also checked for an appearance change by visual observation. Table 3 shows the results.

**Table 3**

| | Weight residual rate (%) after 20 days | Appearance change |
|---|---|---|
| Polypropylene alone (control) | 99.6 | Colored in yellow but not changed in form |
| Silicic acid-treated solid solution of the present invention, contained (Example) | 99.6 | No coloring and no change in form |
| Solid solution not treated with silicic acid, contained (Comparative Example 1) | 98.0 | Aperipheral part was changed to brown. Missing was found in some places, and form was changed. |

## Claims

1. An ultraviolet absorber containing, as active ingredient, a zinc oxide solid solution of the formula (1),
(Zn)₁₋ₓM³⁺_{x-δ}O·nSiO₂ (1)
wherein M³⁺ is at least one trivalent metal selected from Al³⁺, Fe³⁺ and Ce³⁺, 0 < x < 0.2, 0.001 < n < 0.2 and δ is a cationic lattice defect,
in which solid solution the surface of a crystal is chemically bonded to a silicic acid,
which solid solution has an average secondary particle diameter of 1.5 µm or less and a BET specific surface area of at least 40 m²/g.

2. An absorber according to claim 1, wherein M³⁺ is Al³⁺ or a combination of A1³⁺ and Fe³⁺ or A1³⁺ and Ce³⁺.

3. An absorber according to claim 1 or 2, wherein M³⁺ is A1³⁺.

4. An absorber according to any one of the preceding claims, wherein 0.05 < x ≤ 0.12.

5. An absorber according to any one of the preceding claims, wherein 0.005 ≤ n ≤ 0.1.

6. An absorber according to any one of the preceding claims, wherein the solid solution has an average secondary particle diameter of 1.0 µm or less and a BET specific surface area of at least 50 m²/g.

7. An absorber according to any one of the preceding claims, wherein the solid solution has an average secondary particle diameter of 0.5 µm or less and a BET specific surface area of at least 60 m²/g.

8. A process for the production of an ultraviolet absorber as defined in any one of the preceding claims, which process comprises carrying out coprecipitation of a mixed aqueous solution of a water-soluble salt of Zn and a water-soluble salt of at least one trivalent metal selected from Al³⁺, Fe³⁺ and Ce³⁺ while maintaining a pH value of at least 6 with an alkali aqueous solution to obtain a coprecipitate, mixing the coprecipitate with a silicic acid or a silicic acid precursor before or after washing the coprecipitate with water or an alkali metal carbonate aqueous solution, drying the mixture, and firing the dried mixture at 300°C or higher.

9. A process according to claim 8, wherein the coprecipitate is pulverized in an aqueous medium with a wet ball mill or the fired mixture is pulverized in an organic medium with a wet ball mill.

10. A composition suitable as a sun screen, comprising a zinc oxide solid solution as defined in any one of claims 1 to 7 and a carrier or diluent.

11. A composition according to claim 10, wherein the solid solution is surface-treated with perfluoroalkyl phosphate ester and/or a silicone oil.

12. A resin composition containing 100 parts by weight of a resin and 0.01 to 10 parts by weight of a zinc oxide solid solution as defined in any one of claims 1 to 7.

13. Use of a composition as defined in claim 12 as a packaging material.

14. Non-therapeutic use of a composition according to claim 10 or 11, to prevent pigmentation of human skin.

15. Non-therapeutic use of a zinc oxide solid solution as defined in any one of claims 1 to 7, in absorbing ultraviolet rays.

16. A zinc oxide solid solution as defined in any one of claims 1 to 7, for use in treating the human or animal body by therapy.

17. Use of a zinc oxide solid solution as defined in any one of claims 1 to 7, in the manufacture of a medicament for topical use in absorbing ultraviolet rays.
